# EUROPEAN PATENT APPLICATION

(11) **EP 2 072 079 A1**
(43) Date of publication of application: **24.06.2009**
(21) Application number: 07829487.3
(22) Date of filing: 10.10.2007
(51) Int. Cl.: A61N 1/30, A61C 19/06

(54) **ARTIFICIAL TOOTH-TYPE IONTOPHORESIS DEVICE**

(30) Priority: 10.10.2006 JP 2006276548
(71) Applicant: TTI ellebeau, Inc., Tokyo 140-0002 (JP)
(72) Inventor: NAKAYAMA, Mizuo, Tokyo 140-0002 (JP); MATSUMURA, Takehiko, Tokyo 140-0002 (JP); SHIBATA, Tsutomu, Tokyo 140-0002 (JP)
(74) Representative: Liesegang, Eva
(86) International application number: PCT/JP2007/069749
(87) International publication number: WO 2008/044705

(57) **Abstract**

Provided is an iontophoresis device which can be stably applied in the oral cavity of a patient over a long time period and can alleviate unpleasantness of the patient associated with the application thereof in the oral cavity. More specifically, provided is an iontophoresis device which comprises an electric power source, a working electrode assembly, which is connected to the electric power source, for releasing an ionic drug by iontophoresis to administer the drug into a living organism through an oral mucosa, and a non-working electrode assembly as a counter electrode of the working electrode assembly, **characterized in that** the electric power source, the working electrode assembly and the non-working electrode assembly are all placed in a support constituted of a denture.

## Description

### Related Application

The present patent application claims priority based on Japanese Patent Application No. 2006-276548 (filing date: the tenth day of October 2006), previously filed in Japan. All the disclosed contents in the previous patent application are incorporated herein by reference.

### Background of the Invention

### Field of the Invention

The present invention relates to a technique (transdermal drug delivery) for transdermally administering various ionic drugs by iontophoresis, in particular, an iontophoresis device incorporated into a denture.

### Prior Art

Iontophoresis refers to a method involving applying electromotive force to an ionic drug placed on the surface of the skin or the mucosa (hereinafter merely referred to as "skin") of a predetermined region of a living organism, driving the ionic drug to introduce (impregnate) the drug into the living organism through the skin (see JP 63-35266 A).

For example, a positively charged ion is driven (transported) into the skin at an anode side of an electric system of an iontophoresis device. In contrast, a negatively charged ion is driven (transported) into the skin at a cathode side of the electric system of the iontophoresis device.

Conventionally, various proposals have been made on the iontophoresis device described above (for example, JP 63-35266 A, JP 04-297277 A, JP 2000-229128 A, JP 2000-229129 A, JP 2000-237327 A, JP 2000-237328 A, WO 03/037425, JP 2004-518707 A, JP 2004-231575 A, and JP 2003-501379 A).

In order for efficient drug delivery may be realized, it may be desirable for a drug delivery system to administer a drug through an oral mucosa having high absorption efficiency depending on the state of a disease and the nature of the drug. However, when a conventional iontophoresis device is placed on an oral mucosa, a contact surface between the surface of the mucosa and the iontophoresis device becomes unstable, so it may become difficult to administer the drug efficiently over a long time period. Further, when the conventional iontophoresis device is placed in the oral cavity of a patient, the following problem arises: various components of the iontophoresis device such as an electric power source and a cord are exposed in, or outside, the patient's mouth, so the patient must endure the unpleasantness.

Therefore, it is important that an iontophoresis device be stably applicable in the oral cavity of a patient over a long time period and that the unpleasantness of the patient associated with the application of the iontophoresis device in the oral cavity be alleviated.

### Summary of the Invention

The present invention has been made in view of the above-mentioned problems of the prior art, and an object of the present invention is to provide an iontophoresis device having the following characteristics: the iontophoresis device is stably applicable in the oral cavity of a patient over a long time period, and the unpleasantness of the patient associated with the application of the iontophoresis device in the oral cavity can be alleviated.

In order to solve the above-mentioned problems, the present invention provides an iontophoresis device, including: an electric power source; a working electrode assembly for releasing an ionic drug by iontophoresis to administer the drug to a living organism through an oral mucosa, the working electrode assembly being connected to the electric power source; and a non-working electrode assembly as a counter electrode of the working electrode assembly, **characterized in that** the electric power source, the working electrode assembly, and the non-working electrode assembly are all placed in a support constituted of a denture.

Since the iontophoresis device according to the present invention is incorporated into a support constituted of a denture, the iontophoresis device can be stably applied in the oral cavity of a patient over a long time period, and the unpleasantness of the patient associated with the application of the iontophoresis device in the oral cavity can be alleviated.

### Brief Description of the Drawings

Fig. 1 is a view showing the outline of a support composed of a denture which stores an iontophoresis device according to the present invention.
Fig. 2 is a sectional view of an iontophoresis device in a preferred embodiment the working electrode assembly and non-working electrode assembly of which are placed in different artificial teeth.
Fig. 3 is a perspective view of the back surface side of the iontophoresis device the working electrode assembly and non-working electrode assembly of which are placed in different artificial teeth.
Fig. 4 is a sectional view of an iontophoresis device in another preferred embodiment the working electrode assembly and non-working electrode assembly of which are placed in different artificial teeth.
Fig. 5 is a sectional view of an iontophoresis device the working electrode assembly and non-working electrode assembly of which are placed in the same artificial tooth.
Fig. 6 is a sectional view of an iontophoresis device the working electrode assembly and non-working electrode assembly of which are placed in a denture base.

### Specific Description of the Invention

The term "front surface" as used herein refers to a side near the skin of a living organism on the path of electric current flowing through the inside of the electrode assembly in administering the ionic drug.
In addition, the phrase "selectively allows an ion to pass through" as used herein does not mean that no restrictions are placed on the passage of ions; instead, the phrase includes the case where ions to be selectively allowed to pass through are allowed to pass through at a sufficiently high rate or in a sufficiently large amount as compared to ions opposite in polarity to the above ions even when the passage of each ion is restricted to some extent.

As described above, an iontophoresis device according to the present invention is **characterized in that** its electric power source, working electrode assembly, and non-working electrode assembly are all placed in a support constituted of a denture.

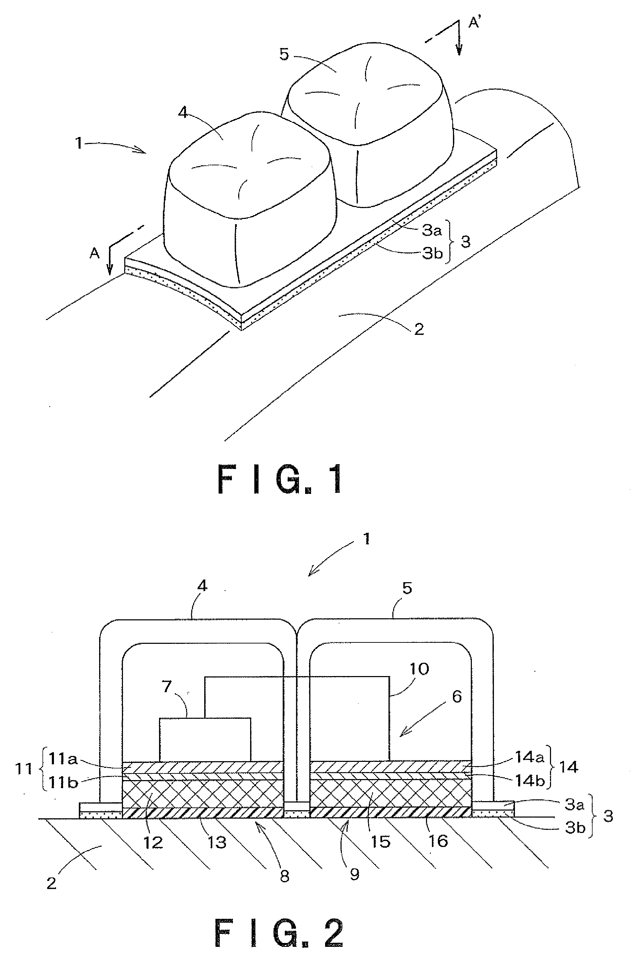

Hereinafter, the present invention will be described on the basis of specific preferable examples shown in the drawings.
An embodiment shown in Fig. 1 corresponds to a state where a support 1 constituted of a denture into which the iontophoresis device according to the present invention is incorporated is placed on a gingival mucosa 2.
In Fig. 1, the support 1 is composed of a denture base 3 and artificial teeth (4 and 5). The denture base 3 is constituted of a base material 3a and an adhesive layer 3b for bonding the gingival mucosa 2 and the support 1, the adhesive layer being placed on the front surface of the base material. The support 1 is stably fixed on the gingival mucosa (oral mucosa) 2 by the adhesive layer 3b. Further, the support 1 is used as a denture when the iontophoresis device is used or is not used.

Fig. 2 is a sectional view of the support 1 in Fig. 1 taken along the line A-A'.
The support 1 stores an iontophoresis device 6 in itself. The iontophoresis device 6 includes: an electric power source 7; a working electrode assembly 8 for releasing an ionic drug by iontophoresis to administer the drug to a living organism through the oral mucosa, the working electrode assembly being connected to the electric power source 7; and a non-working electrode assembly 9 as the counter electrode of the working electrode assembly 8. The working electrode assembly 8 and the non-working electrode assembly 9 are placed in different artificial teeth; the working electrode assembly 8 is stored in the artificial tooth 4, and the non-working electrode assembly 9 is stored in the artificial tooth 5. Further, the electric power source 7 is directly connected to the working electrode assembly 8 in the artificial tooth 4, and is connected to the non-working electrode assembly 9 through a cord 10.

In addition, the working electrode assembly 8 includes: an electrode portion 11 connected to the side of the electric power source 7 identical in polarity to the ionic drug; a drug solution holding portion 12 for holding the ionic drug, the drug solution holding portion being placed on the front surface of the electrode portion 11; and an ion exchange membrane 13 that selectively allows an ion identical in polarity to the ionic drug to pass through itself, the ion exchange membrane being placed on the front surface of the drug solution holding portion 12. Further, the above electrode portion 11 is constituted of: a collector 11a connected to the side of the electric power source 7 identical in polarity to the ionic drug; and a polarizable electrode 11b placed on the front surface of the collector 11a.

In addition, the non-working electrode assembly 9 includes: an electrode portion 14 connected to the side of the electric power source 7 opposite in polarity to the working electrode assembly 8; an electrolyte solution holding portion 15 for holding an electrolyte solution, the electrolyte solution holding portion being placed on the front surface of the electrode portion 14; and an ion exchange membrane 16 that selectively allows an ion opposite in polarity to the ionic drug to pass through itself, the ion exchange membrane being placed on the front surface of the electrolyte solution holding portion 15. Further, the above electrode portion 14 is constituted of a collector 14a and a polarizable electrode 14b placed on the front surface of the collector 14a.

In the working electrode assembly 8 of Fig. 2, the drug solution in the drug solution holding portion 12 contacts the polarizable electrode 11b. Accordingly, the entirety, or at least part, of energization from the polarizable electrode 11b to the drug solution holding portion 12 is generated by the formation of an electric double layer as a result of the trapping of an ion opposite in polarity to the ionic drug in the drug solution holding portion 12 in the polarizable electrode 11b. Therefore, energization to the drug solution can be performed in a state where an electrode reaction such as electrolysis is prevented from occurring, or is reduced. As a result, energization sufficient for the administration of a required amount of the ionic drug can be performed in a state where the generation of a gas or undesirable ion is suppressed, or is at least reduced.
Further, upon energization, the ionic drug in the drug solution holding portion 12 in the working electrode assembly 8 moves by electrophoresis toward the side opposite to the electrode portion 11 with an electric field, and is transdermally administered to the living organism through the ion exchange membrane 13. In this case, the ion exchange membrane 13 placed on the gingival mucosa 2 can efficiently release the ionic drug because the membrane selects an ion identical in polarity to the ionic drug.

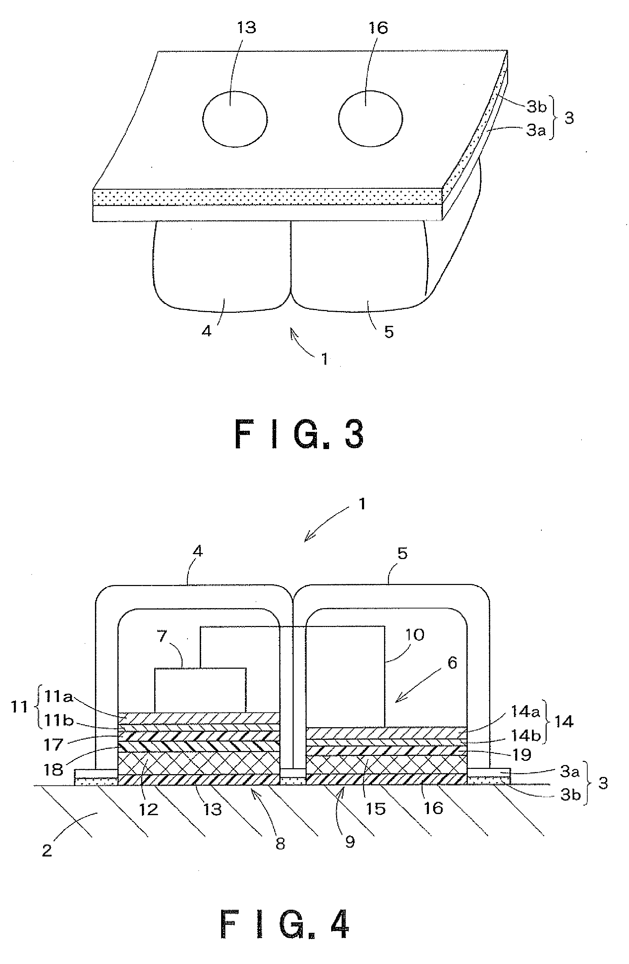

In addition, Fig. 3 is a perspective view of the back surface side of the iontophoresis device in Fig. 2.
The outer periphery of each of the ion exchange membrane 13 of the working electrode assembly 8 and the ion exchange membrane 16 of the non-working electrode assembly 9 is surrounded by the adhesive layer 3b at a contact surface between the support 1 and the gingival mucosa 2.

Fig. 4 is a sectional view of an iontophoresis device according to another preferred embodiment of the present invention.
In Fig. 4, the support 1 is of the same constitution as that of Fig. 2, and the iontophoresis device 6 has the same constitution as that of Fig. 2 except the constitution of each of both the electrode assemblies (8 and 9).
The working electrode assembly 8 includes: the collector 11a connected to the side of the electric power source 7 identical in polarity to an ionic drug in the drug solution holding portion 12; the polarizable electrode 11b placed on the front surface of the collector 11a; a separator 17 for holding an electrolyte solution, the separator being placed on the front surface of the polarizable electrode 11b; an ion exchange membrane 18 that selectively allows an ion opposite in polarity to the ionic drug to pass through itself, the ion exchange membrane being placed on the front surface of the separator 17; the drug solution holding portion 12 for holding the ionic drug, the drug solution holding portion being placed on the front surface of the ion exchange membrane 18; and the ion exchange membrane 13 that selectively allows an ion identical in polarity to the ionic drug to pass through itself, the ion exchange membrane being placed on the front surface of the drug solution holding portion 12. Although not shown, a drug solution identical in composition to the ionic drug in the drug solution holding portion is applied as a skin contacting layer to a contact surface between the working electrode assembly 8 and the gingival mucosa 2 as desired.

In addition, the non-working electrode assembly 9 includes: the collector 14a connected to the side of the electric power source 7 opposite in polarity to the working electrode assembly 8; the polarizable electrode 14b placed on the front surface of the collector 14a; a separator 19 for holding an electrolyte solution, the separator being placed on the front surface of the polarizable electrode 14b; the electrolyte solution holding portion 15 for holding the electrolyte solution, the electrolyte solution holding portion being placed on the front surface of the separator 19; and the ion exchange membrane 16 that selectively allows an ion opposite in polarity to the ionic drug to pass through itself, the ion exchange membrane being placed on the front surface of the electrolyte solution holding portion 15. Although not shown, an electrolyte solution identical in composition to the electrolyte solution in the electrolyte solution holding portion is applied as a skin contacting layer to a contact surface between the non-working electrode assembly 9 and the gingival mucosa 2 as desired.

When the electrolyte solution in the separator 17 contacts the polarizable electrode 11b in the working electrode assembly 8 of Fig. 4, the entirety, or at least part, of energization from the polarizable electrode 11b to the separator 17 is generated by the formation of an electric double layer as a result of the trapping of an ion in the separator 17 opposite in polarity to the ionic drug in the polarizable electrode 11b. Therefore, energization into the working electrode assembly 7 can be performed in a state where an electrode reaction is prevented from occurring, or is reduced. As a result, energization sufficient for the administration of a required amount of the ionic drug can be performed in a state where the generation of a gas such as hydrogen or chlorine or of an ion undesirable for safety associated with electrolysis is suppressed, or is at least reduced.

Further, in the working electrode assembly 8 of Fig. 4, upon energization, the ionic drug in the drug solution holding portion 12 moves by electrophoresis toward the side opposite to the electrode with an electric field, and is transdermally administered to the living organism through the ion exchange membrane 13. In this case, the ion exchange membrane 18 placed on the electrode side prevents the movement of the ionic drug to the electrode side because the membrane selects an ion opposite in polarity to the ionic drug; meanwhile, the ion exchange membrane 13 placed on the gingival mucosa 2 efficiently releases the ionic drug because the membrane selects an ion identical in polarity to the ionic drug. As a result, the ionic drug can be administered into the gingival mucosa 2 with high efficiency.

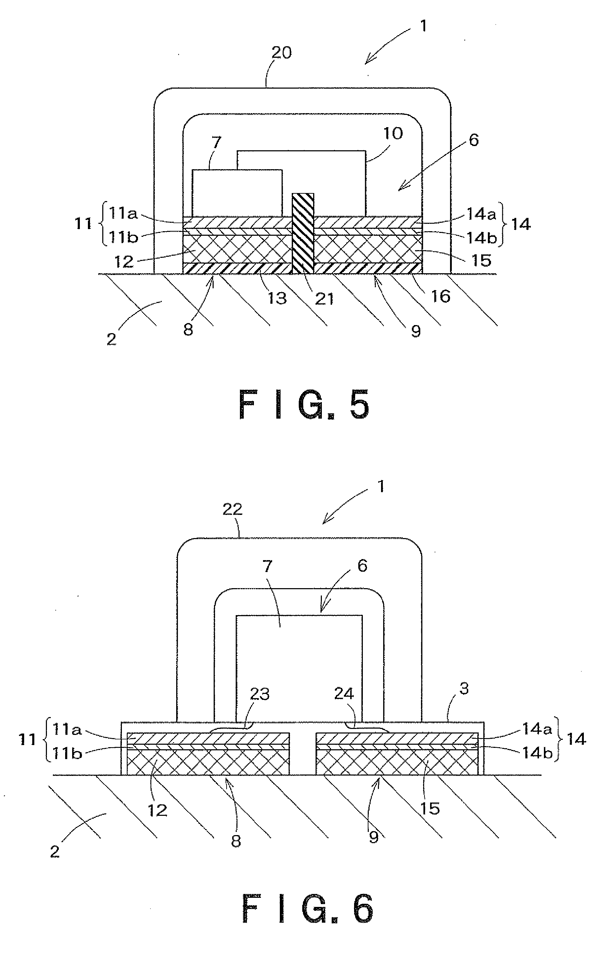

In addition, Fig. 5 is a sectional view of an iontophoresis device according to another preferred embodiment of the present invention.
In Fig. 5, the support 1 is composed of one artificial tooth 20, and the iontophoresis device 6 is stored in the artificial tooth 20. Further, the artificial tooth 20 is connected to an adjacent natural tooth through a clasp composed of a metal such as titanium, though not shown. The constitution of each of both the electrode assemblies (8 and 9) in the iontophoresis device 6 of Fig. 5 is identical to that of Fig. 1, and, furthermore, both the electrode assemblies (8 and 9) are separated from each other by an insulator 21. As described above, the iontophoresis device 6 is integrally constituted, so the device can be advantageously utilized in a narrow space in a mouth.

In addition, Fig. 6 is a sectional view of an iontophoresis device according to another preferred embodiment of the present invention.
In Fig. 6, the support 1 is constituted of one artificial tooth 22 and the denture base 3. The electric power source 7 in the iontophoresis device 6 is stored in the artificial tooth 22, and the working electrode assembly 8 and the non-working electrode assembly 9 in the device are stored in the denture base 3. In addition, both the electrode assemblies (8 and 9) are connected to the electric power source 7 thorough cords (23 and 24).

In addition, the working electrode assembly 8 includes: an electrode portion 11 connected to the side of the electric power source 7 identical in polarity to the ionic drug; and a drug solution holding portion 12 for holding the ionic drug, the drug solution holding portion being placed on the front surface of the electrode portion 11. Further, the above electrode portion 11 is constituted of: a collector 11a; and a polarizable electrode 11b placed on the front surface of the collector 11a.

In addition, the non-working electrode assembly 9 includes: an electrode portion 14 connected to the side of the electric power source 7 opposite in polarity to the working electrode assembly 8; and a electrolyte solution holding portion 15 for holding the electrolyte solution, the electrolyte solution holding portion being placed on the front surface of the electrode portion 14. Further, the above electrode portion 14 is constituted of: a collector 14a; and a polarizable electrode 14b placed on the front surface of the collector 14a.

In the above iontophoresis device 6, a large part of a contact surface between the denture base 3 and the gingival mucosa 2 is constituted of the drug solution holding portion 13 and the electrolyte solution holding portion 16, so the ionic drug can be efficiently administered with the aid of the wide area.

In addition, according to another preferred embodiment of the present invention, the above support is constituted of an implant structure mounted with an artificial tooth. In such embodiment, the electric power source, the working electrode assembly, and the non-working electrode assembly have only to be stored in any part in the artificial tooth and/or an implant. When the above implant structure is used as a support, the iontophoresis device can be stably placed on the gingival mucosa, whereby the ionic drug can be administered with improved efficiency. In the above implant structure, the artificial tooth and the implant are preferably constituted so as to be detachable in consideration of charging and replenishment with the ionic drug. A specific constitution of such implant structure is, for example, the above embodiment shown in any one of Figs. 1 to 6 in which a denture is mounted on at least one implant.

In addition, at least one of the electric power source, the working electrode assembly, and the non-working electrode assembly in the above iontophoresis device is preferably constituted to be detachable from the support constituted of a denture. According to such embodiment, charging, the exchange of each electrode assembly, and the like can be easily performed. In such embodiment, both the electrode assemblies may be integrally produced by, for example, compression molding. In addition, a connecting portion between each electrode assembly and the electric power source can be appropriately provided with fitting portions (such as male and female connectors) each composed of a material such as a metal or conductive silicon rubber. Further, each electrode assembly and the support may be fixed with a removal clasp when they are not used.

The support in the present invention can be produced by, for example, such known denture production method as described below: when the support is to be made from an acrylic resin, the support is produced by a method involving filling a molding tool having a desired morphology with a known base material stuff such as dough in which a polymer powder and a monomer liquid are mixed and polymerizing the stuff by means of, for example, room-temperature polymerization, heat polymerization, or photopolymerization; when the support is to be made from a polyether sulfone-based resin, a polysulfone-based resin, a polycarbonate-based resin, or a polyamide-based resin, the support is produced by a method involving molding with an injection device utilizing the thermoplasticity of any such resin; or when the support is to be made from a silicone-based resin, the support is produced by a method involving filling a molding tool with a mixture of a monomer liquid and a catalyst and polymerizing the mixture by means of, for example, room-temperature polymerization, heat polymerization, or photopolymerization.
Further, the adhesive layer for bonding the above support and the gingival mucosa is not particularly limited as long as the iontophoresis device can be stably placed, and is preferably formed of, in view of preventing the ionic drug from being leaked: an adhesive material formed of a cyanoacrylate monomer and the like; a pressure-adhesive material formed of hydroxypropylmethyl cellulose, ethyl cellulose, gum arabic, gum karaya, sodium alginate, sodium polyacrylate, and the like; an adsorbent material formed of a soft silicone resin and the like; and a waterproof rubber sheet.

Further, in the case where the support is constituted of an implant structure, known implants of a blade-type, a screw-type, a cylinder-type, a hollow-type, and the like can be used as the implant. Further, preferable examples of a component of the implant are ceramics containing apatite as a main component, titanium, cobalt, chromium, a tungsten-molybdenum alloy, alumina, and zirconia. The above implant structure can be produced by: embedding the implant in an alveolar bone with, for example, a gingival punch, a round bur, a spiral drill, and a depth measuring gauge; and mounting the tooth crown portion with an artificial tooth in which the iontophoresis device is incorporated.

Examples of the ionic drug include topical anesthetics (procaine hydrochloride, lidocaine hydrochloride, and the like), therapeutic drugs for gastrointestinal disorders (carnitine chloride and the like), skeletal muscle relaxants (pancuronium bromide and the like), antibiotics (tetracycline-based formulations, kanamycin-based formulations, and gentamicin-based formulations), vitamins (riboflavin phosphate, nicotinic acid, ascorbic acid, folic acid, and the like), adrenocortical hormones (hydrocortisone-based aqueous formulations, dexamethasone-based and prednisolone-based aqueous formulations such as prednisolone sodium phosphate and dexamethasone sodium phosphate, and the like), antibacterial drugs (quinolone-based formulations), vaccines, adjuvants, analgesics (non-steroidal anti-inflammatory analgesic, opioid-based formulations, and the like), vasoactive drugs (vasodilator such as nitroglycerine, and vasoconstrictor), anticoagulants (warfarin, ticlopidine, and the like), antihypertensive drugs (angiotensin converting enzyme inhibitor, angiotensin II receptor antagonist, calcium antagonist, beta blocker, alpha blocker, and antihypertensive diuretic), and insulin. Further, a plurality of those ionic drugs can be used appropriately in combination in accordance with the kind of a disease and the condition of a patient.

The amount of any one of the ionic drugs is determined for each ionic drug in order that a preset, effective blood concentration may be obtained over an effective time period upon application of the drug to a patient; the amount is set by one skilled in the art depending on, for example, the size and thickness of the drug solution holding portion or the like, the area of a drug releasing surface, a voltage in an electrode device, and the time period for which the drug is administered.

In addition, the above drug solution holding portion is constituted of a thin-film body capable of holding the above ionic drug. Such thin-film body preferably has: a sufficient ability to hold the ionic drug; and a sufficient ability to cause the held ionic drug to migrate toward a skin side under a predetermined electric field condition (ionic transferability or ionic conductivity). A material for the thin-film body of which the drug solution holding portion is constituted is, for example, a PP nonwoven fabric, a hydrogel form of an acrylic resin (acrylic hydrogel film), a segmented polyurethane-based gel film, or an ion conductive porous sheet for the formation of a gel-like solid electrolyte disclosed in JP 11-273452 A; the PP nonwoven fabric is preferable.

Further, the drug solution holding portion can be easily caused to hold the ionic drug by, for example, immersing the above thin-film body in the drug solution containing the ionic drug. The term "drug solution" as used herein means a liquid medium prepared by dissolving or suspending the ionic drug in an electrolyte solution to be described later.

The electrode portion in each electrode assembly, which may be obtained by using an inert electrode composed of one kind of a conductive material such as carbon, is preferably constituted of a collector and a polarizable electrode.
A component for the collector used in the electrode assembly is, for example, any one of the conductive materials including metals such as carbon, gold, platinum, silver, copper, and zinc; the component is preferably carbon. In addition, the above collector is preferably a printed electrode obtained by printing a mixture of the above conductive material and an adhesive onto a polyethylene terephthalate (PET) material.

In addition, the polarizable electrode in the present invention is mainly constituted of a conductive base material composed of activated carbon, or preferably carbon fibers or carbon fiber paper. When only activated carbon fibers are used, it is recommended that a layer be formed by combining cloth and felt each composed of activated carbon fibers. In addition, the polarizable electrode can be constituted of activated carbon fibers impregnated with a binder polymer; in this case, activated carbon fibers obtained by carbonizing and activating novoloid fibers which have an extremely large specific surface area (for example, 1,000 to 2,500 m²/g) and a high tensile strength (for example, 300 to 400 N/mm²), and which are excellent in flexibility can be particularly preferably used as the activated carbon fibers. The activated carbon fibers obtained by carbonizing and activating novoloid fibers are available from, for example, Nippon Kynol Inc. under the tradename "Kynol activated carbon fiber".

In addition, the polarizable electrode can be caused to hold an electrolyte solution by being impregnated with the electrolyte solution. Such electrolyte solution in the polarizable electrode is, for example, an electrolyte solution identical in composition to an electrolyte solution held by a separator or electrolyte solution holding portion to be described later.

In addition, the electrolyte solution holding portion in the present invention can be constituted of a thin-film body having the following property: the thin-film body holds an electrolyte solution by being impregnated with the electrolyte solution. A material of the same kind as that used in the above-mentioned drug solution holding portion can be used in he thin-film body.
In addition, the separator in the present invention is not particularly limited as long as the separator can separate each electrode portion and any other member such as the electrolyte solution holding portion or ion exchange membrane, and does not inhibit energization; the separator is preferably constituted of a thin-film body of the same kind as that used in the electrolyte solution holding portion. To be specific, the separator is, for example, a thin-film body having a thickness about one tenth of that of the electrolyte solution holding portion.

In addition, a desired one can be appropriately used as the above electrolyte solution depending on conditions such as a drug to be applied, but an electrolyte solution that does damage to the skin of a living organism by an electrode reaction is preferably avoided. Suitable examples of the above electrolyte solution include aqueous media each containing an electrolyte that is more readily oxidized or reduced in an electrolysis reaction (oxidation at an anode or reduction at a cathode) than water is such as electrolyte solutions each containing: NaCl; a pharmaceutical agent such as ascorbic acid (vitamin C) or sodium ascorbate; or an organic acid such as lactic acid, oxalic acid, malic acid, succinic acid, or fumaric acid and/or a salt of the acid. In addition, a combination of multiple kinds of electrolytes may be added to the above electrolyte solution so that the electrolyte solution may serve as a buffer electrolyte solution; in this case, a fluctuation in pH of the electrolyte solution during energization can be suppressed. Further, a drug solution identical in composition to the drug solution in the above-mentioned drug solution holding portion may be used as the above electrolyte solution, and such embodiment is also included in the present invention.

In addition, a thickening agent is preferably added to the above electrolyte solution. The use of such thickening agent can: prevent a change in property of the iontophoresis device at each of the polarizable electrode, the separator, and the above-mentioned drug solution holding portion over time; and improve the property with which the drug solution holding portion holds the drug. The thickening agent may be added at a content of, for example, 2 wt% or more to the electrolyte solution. Examples of such thickening agent include tacky materials such as hydroxypropylcellulose (HPC) (such as an H-Type manufactured by NIPPON SODA CO., LTD.) and a METOLOSE obtained by chemically treating a water-insoluble cellulose so that the cellulose may be a water-soluble polymer (such as a 90SH-10000SR manufactured by Shin-Etsu Chemical Co., Ltd.).

In addition, as the ion exchange membrane used for the electrode assembly, it is preferable to use a cation exchange membrane and an anion exchange membrane in combination. As the cation exchange membrane, NEOSEPTA CM-1, CM-2, CMX, CMS, CMB, and CLE04-2 manufactured by Tokuyama Corporation, and the like are preferably mentioned. In addition, as the anion exchange membrane, NEOSEPTA AM-1, AM-3, AMX, AHA, ACH, ACS, ALE04-2, and AIP-21 manufactured by Tokuyama Corporation, and the like are preferably mentioned. Further, as another preferable example, a cation exchange membrane in which an ion exchange resin having a cation exchange function is impregnated in a part or whole of pores of a porous film or an anion exchange membrane in which an ion exchange resin having an anion exchange function is impregnated is mentioned.

In addition, a fluorine-based ion exchange resin obtained by introducing an ion exchange group into a perfluorocarbon skeleton or a hydrocarbon-based ion exchange resin using an unfluorinated resin as a skeleton can be used as the above ion exchange resin; the hydrocarbon-based ion exchange resin is preferably used because the resin can be produced by a simple step.

In addition, the ion exchange group which the above ion exchange resin has is not particularly limited as long as the ion exchange group is a functional group which produces a group having negative or positive charge in an aqueous solution. Such functional group may be present in the form of a free acid or salt.
Examples of the cation exchange group include a sulfonic acid group, a carboxylic acid group, and a phosphonic acid group, and preferred is a sulfonic acid group. Further, as a counter cation for the cation exchange group, there are exemplified: alkali cations such as a sodium ion and a potassium ion; and an ammonium ion. In addition, examples of the anion exchange group include a primary amino group, a secondary amino group, a tertiary amino group, a quaternary ammonium group, a pyridyl group, an imidazole group, a quaternary pyridium group, and a quaternary imidazolium group, and preferred is a quaternary ammonium group or a quaternary pyridium group. Further, as a counter cation for the anion exchange group, there are exemplified: halogen ions such as a chlorine ion; and a hydroxy ion.

In addition, in each electrode assembly according to the present invention, a skin contacting layer can be provided for a contact surface between the electrode assembly and an oral mucosa as desired. Such skin contacting layer may be constituted by applying the above electrolyte solution or drug solution to the above contact surface. Further, a carrier such as a PP nonwoven fabric in a dry state, or a product obtained by impregnating the above carrier with the electrolyte solution or drug solution can also be used as the skin contacting layer. The amount of the electrolyte solution or drug solution used in the above skin contacting layer is appropriately adjusted by one skilled in the art depending on, for example, the size of the contact surface between the electrode assembly and the oral mucosa.

In addition, when each electrode assembly according to the present invention is used, for example, the following conditions are adopted as preferable energization conditions in the iontophoresis device: a constant current, specifically, 0.1 to 0.5 mA/cm², or preferably 0.1 to 0.3 mA/cm² is flowed, and a safe voltage at which the above constant current is realized, specifically, 50 V or less, or preferably 30 V or less is applied.
In addition, the electric power source is preferably a chargeable battery in consideration of continuous use of the iontophoresis device.

In addition, in the iontophoresis device according to the present invention, such embodiment as described below is also permitted, and is also included in the present invention: both the electrode assemblies are each caused to hold the ionic drug, and the ionic drug is administered from each of both the electrode assemblies. Further, the present invention can be implemented even when the total number of electrode assemblies, and a combination of a working electrode assembly and a non-working electrode assembly are appropriately changed. One skilled in the art will easily hit upon a constitution for the implementation from the above specific examples.

Details of each of the above-mentioned component are disclosed in WO 03/037425 filed by the applicant of the present invention, and all the disclosed contents in this document are incorporated herein by reference.

## Claims

1. An iontophoresis device, comprising:
an electric power source;
a working electrode assembly for releasing an ionic drug by iontophoresis to administer the drug to a living organism through an oral mucosa, the working electrode assembly being connected to the electric power source; and
a non-working electrode assembly as a counter electrode of the working electrode assembly,
**characterized in that** the electric power source, the working electrode assembly, and the non-working electrode assembly are all placed in a support constituted of a denture.

2. An iontophoresis device according to claim 1, **characterized in that** the working electrode assembly and the non-working electrode assembly are placed in different artificial teeth in the denture.

3. An iontophoresis device according to claim 1, **characterized in that** the working electrode assembly and the non-working electrode assembly are placed in the same artificial teeth in the denture.

4. An iontophoresis device according to claim 1, **characterized in that** both the working electrode assembly and the non-working electrode assembly are placed in a denture base in the denture.

5. An iontophoresis device according to claim 1, **characterized in that** the denture comprises an implant structure mounted with an artificial tooth.

6. An iontophoresis device according to claim 1, **characterized in that** at least one of the electric power source, the working electrode assembly, and the non-working electrode assembly is constituted to be detachable from the support.

7. An iontophoresis device according to claim 1, **characterized in that** the working electrode assembly comprises:
an electrode portion connected to a side of the electric power source identical in polarity to the ionic drug; and
a drug solution holding portion for holding the ionic drug, the drug solution holding portion being placed on a front surface of the electrode portion.

8. An electrode assembly according to claim 7, **characterized in that** the electrode portion comprises:
a collector connected to the side of the electric power source identical in polarity to the ionic drug; and
a polarizable electrode placed on a front surface of the collector.

9. An iontophoresis device according to claim 7, **characterized in that** the working electrode assembly further comprises an ion exchange membrane that selectively allows an ion identical in polarity to the ionic drug to pass through itself, the ion exchange membrane being placed on a front surface of the drug solution holding portion.

10. An iontophoresis device according to claim 1, wherein the working electrode assembly comprises:
a collector connected to a side of the electric power source identical in polarity to the ionic drug;
a polarizable electrode placed on a front surface of the collector;
a separator for holding an electrolyte solution, the separator being placed on a front surface of the polarizable electrode;
an ion exchange membrane that selectively allows an ion opposite in polarity to the ionic drug to pass through itself, the ion exchange membrane being placed on a front surface of the separator;
a drug solution holding portion for holding the ionic drug, the drug solution holding portion being placed on a front surface of the ion exchange membrane; and
an ion exchange membrane that selectively allows an ion identical in polarity to the ionic drug to pass through itself, the ion exchange membrane being placed on a front surface of the drug solution holding portion.

11. An iontophoresis device according to claim 1, **characterized in that** the non-working electrode assembly comprises:
an electrode portion connected to a side of the electric power source opposite in polarity to the working electrode assembly; and
an electrolyte solution holding portion for holding the electrolyte solution, the electrolyte solution holding portion being placed on a front surface of the electrode portion.

12. An electrode assembly according to claim 11, **characterized in that** the electrode portion comprises:
a collector connected to the side of the electric power source opposite in polarity to the working electrode assembly; and
a polarizable electrode placed on a front surface of the collector.

13. An iontophoresis device according to claim 11, **characterized in that** the non-working electrode assembly further comprises an ion exchange membrane that selectively allows an ion opposite in polarity to the ionic drug to pass through itself, the ion exchange membrane being placed on a front surface of the drug solution holding portion.

14. An iontophoresis device according to claim 1, **characterized in that** the non-working electrode assembly comprises:
the collector connected to a side of the electric power source opposite in polarity to the working electrode assembly;
a polarizable electrode placed on a front surface of the collector;
a separator for holding an electrolyte solution, the separator being placed on a front surface of the polarizable electrode;
an electrolyte solution holding portion for holding the electrolyte solution, the electrolyte solution holding portion being placed on a front surface of the separator; and
an ion exchange membrane that selectively allows an ion opposite in polarity to the ionic drug to pass through itself, the ion exchange membrane being placed on a front surface of the electrolyte solution holding portion.

15. An iontophoresis device according to claim 1, wherein the oral mucosa comprises a gingival mucosa.
